# EUROPEAN PATENT SPECIFICATION

(11) **EP 3 100 665 B1**
(45) Date of publication and mention of the grant of the patent: **10.10.2018**
(21) Application number: 15742825.1
(22) Date of filing: 21.01.2015
(51) Int. Cl.: A61B 1/00, G02B 23/26, A61B 5/06, G02B 26/10

(54) **SCANNING ENDOSCOPIC DEVICE AND CONTROL METHOD THEREFOR**
ENDOSKOPISCHE ABTASTVORRICHTUNG UND STEUERUNGSVERFAHREN DAFÜR
DISPOSITIF ENDOSCOPIQUE À BALAYAGE ET SON PROCÉDÉ DE COMMANDE

(30) Priority: 29.01.2014 JP 2014014258
(43) Date of publication of application: 07.12.2016
(73) Proprietor: Olympus Corporation, Tokyo 192-8507 (JP)
(72) Inventor: SUGIMOTO, Naoya, Hachioji-shi, Tokyo 192-8507 (JP)
(74) Representative: Schicker, Silvia
(86) International application number: PCT/JP2015/051580
(87) International publication number: WO 2015/115277

(56) References cited:
- WO-A1-2008/143623
- JP-A- 2010 142 597
- JP-A- 2010 527 688
- JP-A- 2011 125 617
- US-A1- 2010 157 037

## Description

### {Technical Field}

The present invention relates to a scanning endoscope apparatus.

### {Background Art}

There is a known endoscope apparatus that scans illumination light over an observation subject in a spiraling manner by driving a distal-end portion of an optical fiber for guiding the illumination light, that makes the light exit from the distal-end portion of the optical fiber, that detects, in accordance with a predetermined sampling rate, a series of sample pixels from light reflected at the observation subject, and that forms an image by using these sample pixels (for example, see Patent Literature 1).

### {Citation List}

### {Patent Literature}

{PTL 1} Japanese Unexamined Patent Application, Publication No. 2010-142605

### {Summary of Invention}

### {Technical Problem}

With the endoscope apparatus of Patent Literature 1, in the case in which observation is performed while moving the viewing-field region, an image becomes distorted when the moving speed of the viewing-field region is increased relative to the amount of time required for acquiring one image (frame rate). In other words, in order to acquire a low-distortion image, it is necessary to set the moving speed of the viewing-field region sufficiently low relative to the frame rate, which makes the observation time-consuming.

US 2010/0157037 A1 describes an endoscope including a scanning fiber transmitting illumination light and an image fiber transmitting light that is reflected off an observation target. A scanning unit is provided at a tip portion of the endoscope. The scanning unit has a cylindrical actuator and scans the illumination light over the target. A course traced by a scanning beam forms a spiral pattern. When motion of the tip portion is detected by an acceleration sensor, a number of spiral scanning lines is decreased.

The present invention has been conceived in light of the above-described circumstances, and an object thereof is to provide a scanning endoscope apparatus and a control method of the same, with which it is possible to acquire an image in which distortion is suppressed even if the moving speed of a viewing-field region is increased.

### {Solution to Problem}

The present invention provides a scanning endoscope apparatus according to independent claim 1. Preferred embodiments are defined by the dependent claims.

An aspect of the present disclosure is a scanning endoscope apparatus including a scanner that has an optical fiber, which guides light coming from a light source and makes the light exit from an exit end thereof, and a drive portion, which displaces the exit end of the optical fiber in a direction that intersects an optical axis, and that scans the light that is made to exit from the exit end over an observation subject; a light detection portion that receives return light coming from the observation subject, generated by scanning of the light by the scanner; a speed detection portion that detects a moving speed of the scanner; and a control portion that controls the drive portion so as to decrease a length of a scanning track of the light on the observation subject with an increase in the moving speed detected by the speed detection portion.

With this aspect, when the exit end of the optical fiber is displaced in the directions that intersect the optical axis due to the actuation of the drive portion of the scanner in a state in which the exit end of the optical fiber is made to face the observation subject, the light coming from the light source, which has been guided inside the optical fiber and made to exit from the exit end, is two-dimensionally scanned over the observation subject. Then, when the return light returning from the observation subject is received by the light detection portion, an image of the observation subject can be acquired by using the intensity of the return light and positions at which the light has been scanned by the scanner.

In this case, when the scanner is moved relative to the observation subject, the moving speed of the scanner is detected by the speed detection portion. Then, in the case in which the moving speed is increased, the control portion controls the drive portion so as to decrease the length of the scanning track of the light. In other words, without changing the scanning speed of the light due to the drive portion, it is possible to decrease the amount of time required for acquiring one image, and it is possible to enhance the frame rate. As a result, it is possible to acquire an image in which distortion is suppressed even if the moving speed of the scanner is increased.

In the above-described aspect, the control portion may switch control so as to decrease the length of the scanning track when the moving speed detected by the speed detection portion becomes equal to or greater than a predetermined threshold.

By doing so, when the moving speed of the scanner detected by the speed detection portion is increased and becomes equal to or greater than the predetermined threshold, the length of the scanning track is decreased. By doing so, when the moving speed of the scanner is increased, the frame rate is increased stepwise, and thus, it is possible to acquire an image in which distortion is suppressed.

In the above-described aspect, the control portion may control the drive portion so as to decrease a density of the scanning track in a predetermined scanning region with an increase in the moving speed detected by the speed detection portion.

By doing so, when the moving speed of the scanner detected by the speed detection portion is increased, the density of the scanning track is decreased in the predetermined scanning region, and thus, the length of the scanning track is decreased. As a result, when the moving speed of the scanner is increased, it is possible to acquire an image in which distortion is suppressed by increasing the frame rate by decreasing the density of the scanning track instead of decreasing the resolution, without changing the scanning speed of the light due to the drive portion.

In the above-described aspect, the drive portion may cause the exit end to periodically vibrate, and the control portion may control the drive portion so as to change a pitch of the scanning track due to vibrations of the exit end on the basis of the moving speed detected by the speed detection portion.

By doing so, when the moving speed of the scanner detected by the speed detection portion is increased, the pitch of the scanning track is increased, which decreases the length of the scanning track, and thus, the frame rate is increased. By increasing the spaces between the adjacent tracks, it is possible to acquire an image in which distortion is suppressed by increasing the frame rate instead of decreasing the resolution.

In the above-described aspect, the control portion may control the drive portion so as to decrease an area of a scanning region with an increase in the moving speed detected by the speed detection portion.

By doing so, when the moving speed of the scanner detected by the speed detection portion is increased, the control portion decreases the area of the scanning region. By doing so, it is possible to decrease the length of the scanning track, and it is possible to acquire an image in which distortion is suppressed by increasing the frame rate without changing the scanning speed due to the drive portion. In addition, by compensating for the decrease in the length of the scanning track by decreasing the area of the scanning region, it is possible to acquire an image in which distortion is suppressed while maintaining the resolution.

In the above-described aspect, the drive portion may cause the exit end to periodically vibrate, and the control portion may control the drive portion so as not to change a pitch of the scanning track due to the vibrations of the exit end on the basis of the moving speed detected by the speed detection portion.

By doing so, because the length of the scanning track is decreased while maintaining the pitch of the scanning track, the control portion does not need to decrease the resolution of the image even if the moving speed of the scanner is increased.

The above-described aspect may be provided with a direction detection portion that detects moving direction of the scanner, wherein the control portion may control the drive portion so as to change a density of the scanning track in a predetermined scanning region when the moving direction detected by the direction detection portion is a forward direction with respect to the exiting direction in which the light exits from the exit end, and so as to change an area of a scanning region when the moving direction is a backward direction with respect to the exiting direction in which the light exits from the exit end.

By doing so, when the scanner is moved, the moving direction of the scanner is detected by the direction detection portion. When the scanner is moved in the forward direction with respect to the light-exiting direction, because the control portion controls the drive portion so as to decrease the density of the scanning track with an increase in the moving speed detected by the speed detection portion, it is possible to acquire a low-distortion image by enhancing the frame rate without changing the viewing-field region. On the other hand, when the scanner is moved in the backward direction with respect to the light-exiting direction, because the control portion controls the drive portion so as to decrease the area of the scanning region with an increase in the moving speed detected by the speed detection portion, it is possible to acquire a low-distortion image by enhancing the frame rate by restricting the viewing-field region to a necessary region.

The above-described aspect may include a flushing portion that is provided in an outer circumferential portion of the scanner at an intermediate position thereof in a longitudinal direction and that discharges a transparent liquid in a forward direction with respect to the light-exiting direction when the scanner is moved in a backward direction with respect to the light-exiting direction.

By doing so, by discharging the transparent liquid from the flushing portion while moving the scanner in the backward direction with respect to the light-exiting direction, because the exit end of the optical fiber is made to pass through a region in which the discharged transparent liquid is temporarily retained, it is possible to acquire a clear image of the observation subject by temporarily ensuring a sufficient viewing field.

Another aspect of the present disclosure not forming part of the invention is a control method of a scanning endoscope apparatus in which an exit end of an optical fiber is displaced in a direction that intersects an optical axis, light exiting from the exit end is scanned over an observation subject by a scanner, and an image is acquired by receiving return light coming from the observation subject, the control method of a scanning endoscope apparatus including a speed detecting step of detecting a moving speed of the scanner; and a displacement adjusting step of displacing the exit end so as to decrease a length of a scanning track of the light on the observation subject with an increase in the moving speed detected in the speed detecting step.

With this aspect, when the moving speed of the scanner is detected in the speed detecting step, the length of the scanning track of the light is decreased in the displacement adjusting step with an increase in the detected moving speed. By doing so, it is possible to enhance the frame rate without changing the scanning speed of the scanner, and thus, it is
possible to acquire an image in which distortion is suppressed.

### {Advantageous Effects of Invention}

The present invention affords an advantage in that it is possible to provide a scanning endoscope apparatus and a control method of the same, with which it is possible to acquire an image in which distortion is suppressed even if the moving speed of a viewing-field region is increased.

### {Brief Description of Drawings}

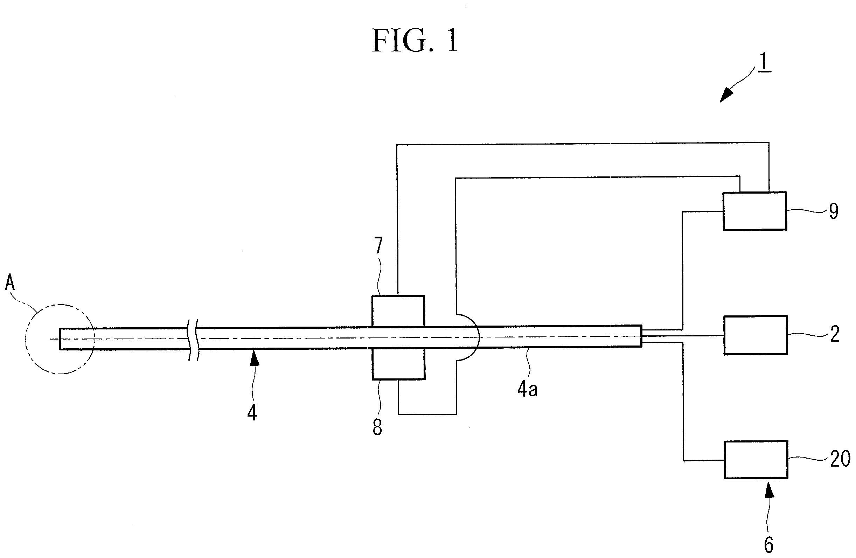

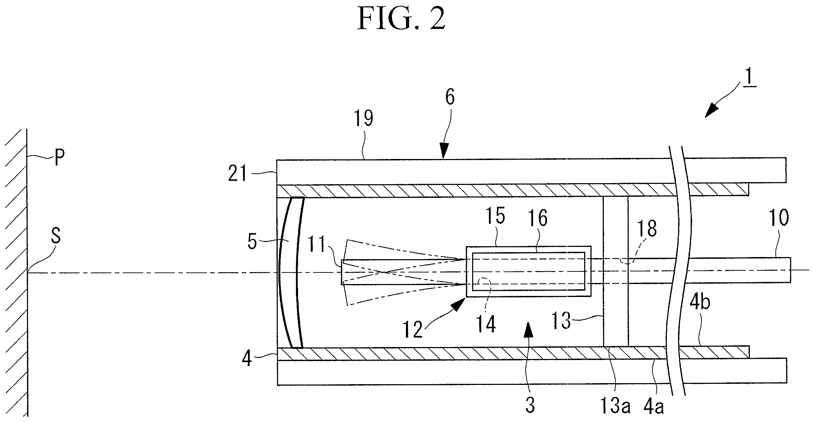

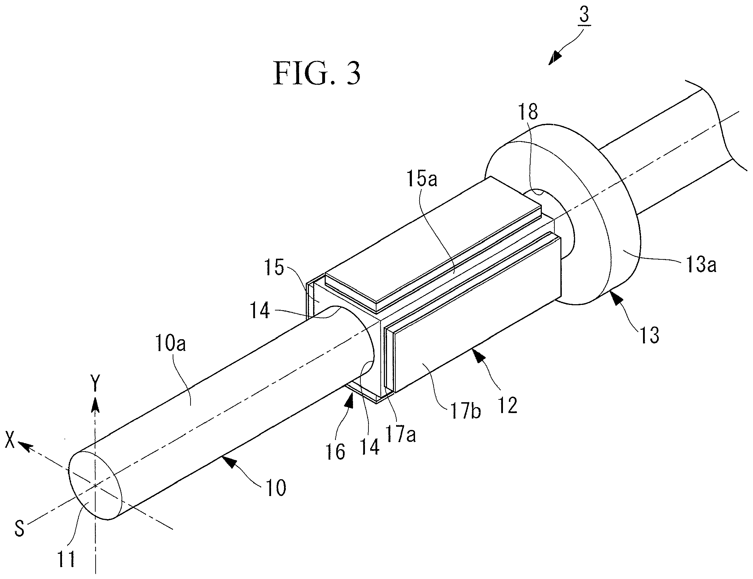

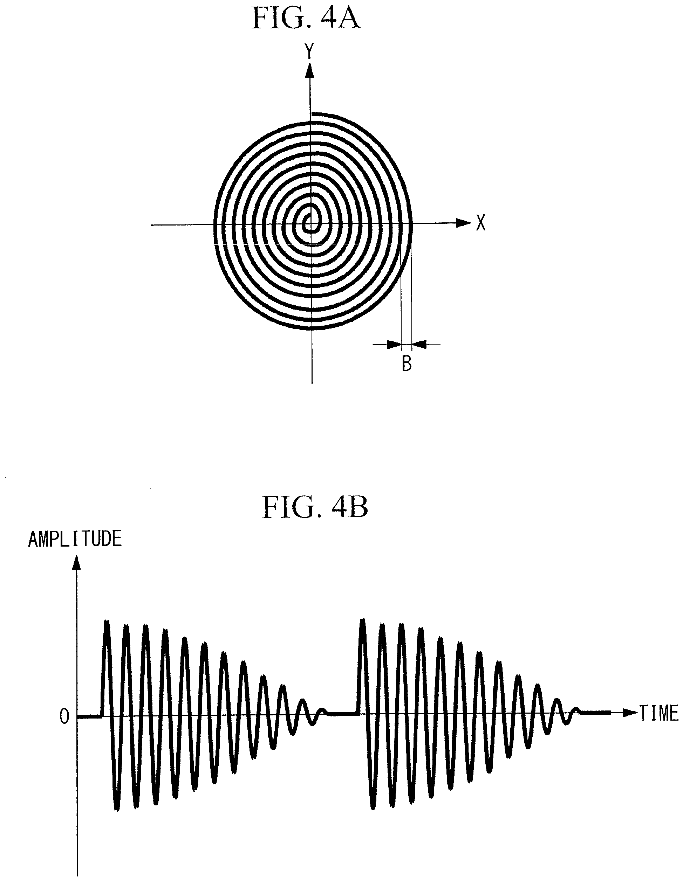

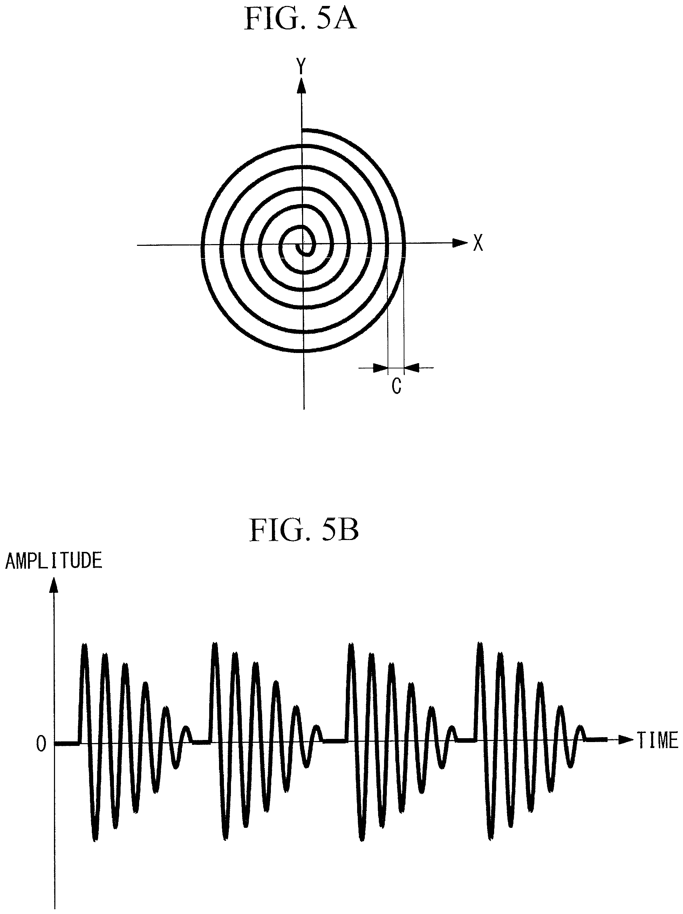

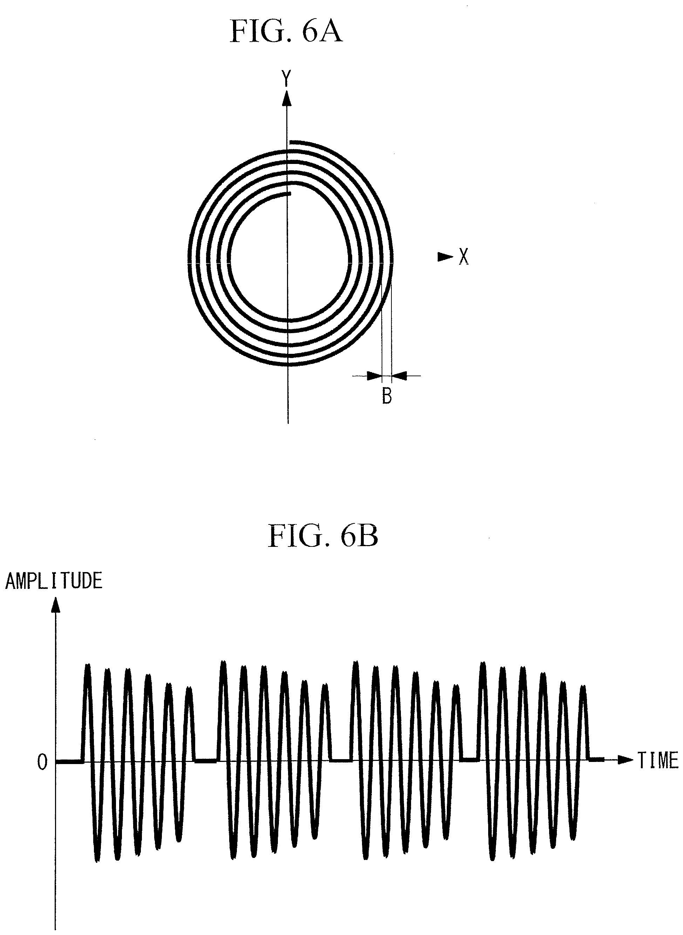

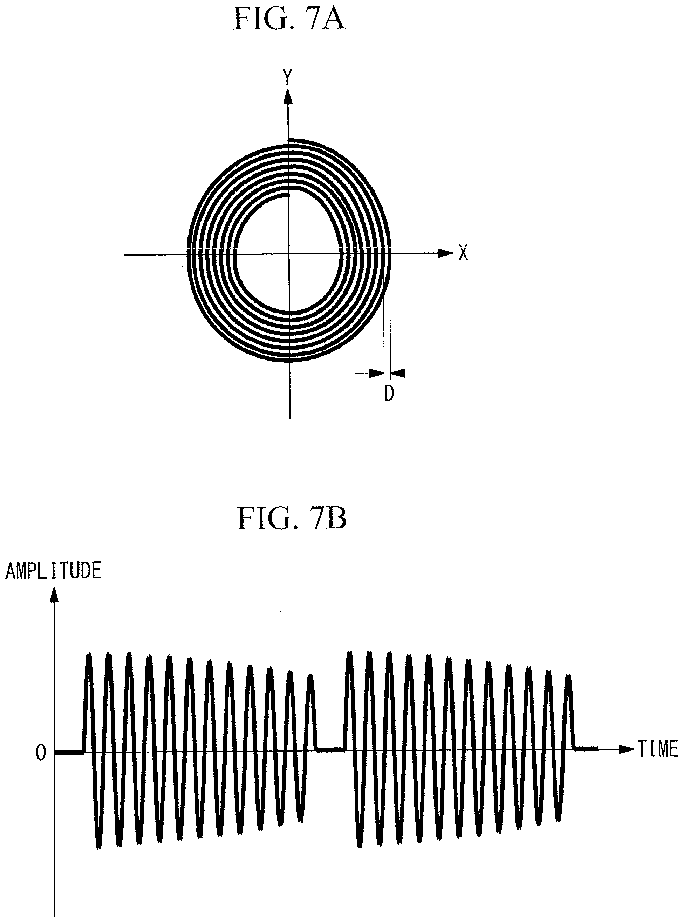

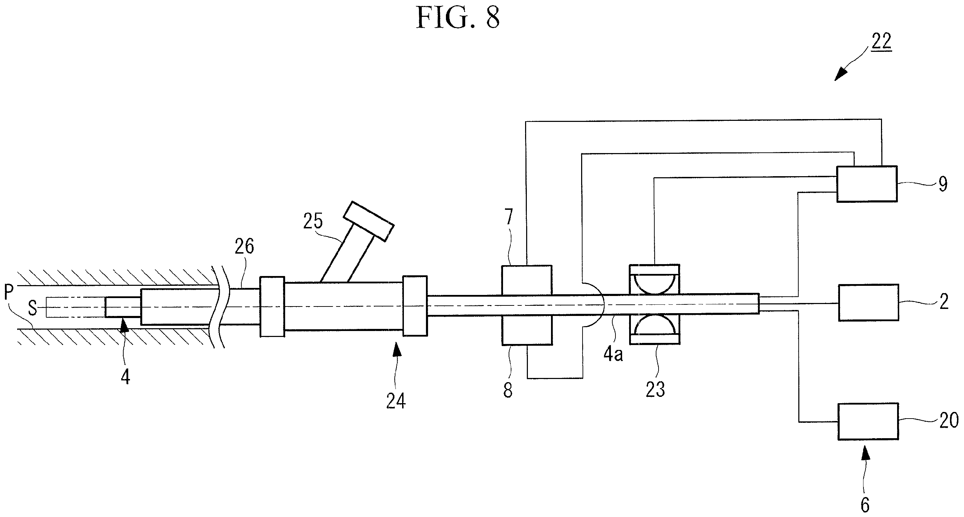

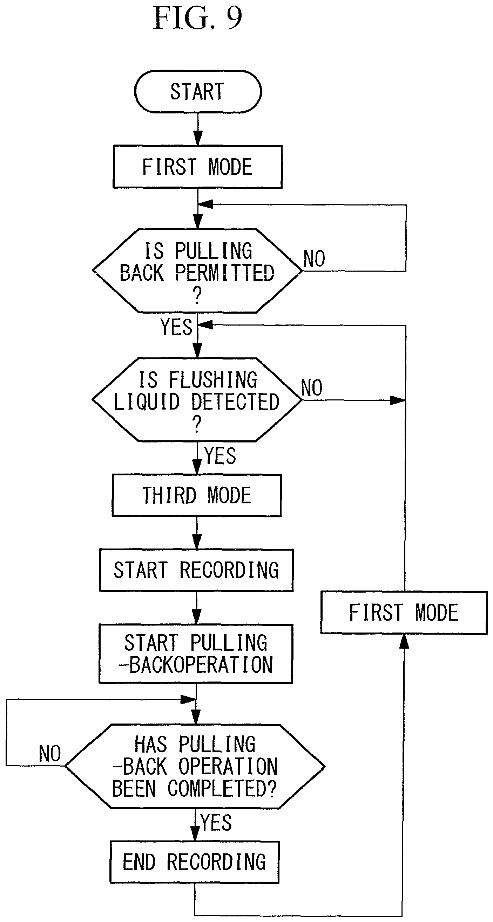

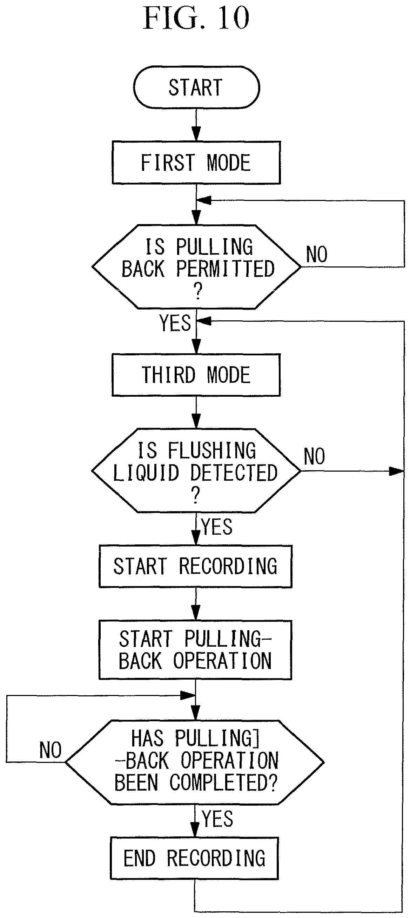

{Fig. 1} Fig. 1 is a schematic diagram of a scanning endoscope apparatus according to a first embodiment of the present invention.
{Fig. 2} Fig. 2 is a longitudinal sectional view of a portion A of the scanning endoscope apparatus in Fig. 1.
{Fig. 3} Fig. 3 is a perspective view showing a scanner of the scanning endoscope apparatus in Fig. 1.
{Fig. 4A} Fig. 4A is a diagram showing scanning tracks of illumination light in the case in which a control portion of the scanning endoscope apparatus in Fig. 1 controls an actuator in a first mode.
{Fig. 4B} Fig. 4B is a diagram showing voltage signals applied to a drive portion in the case in which the control portion of the scanning endoscope apparatus in Fig. 1 controls the actuator in the first mode.
{Fig. 5A} Fig. 5A is a diagram showing scanning tracks of the illumination light in the case in which the control portion of the scanning endoscope apparatus in Fig. 1 controls the actuator in a second mode.
{Fig. 5B} Fig. 5B is a diagram showing voltage signals applied to the drive portion in the case in which the control portion of the scanning endoscope apparatus in Fig. 1 controls the actuator in the second mode.
{Fig. 6A} Fig. 6A is a diagram showing scanning tracks of the illumination light in the case in which the control portion of the scanning endoscope apparatus in Fig. 1 controls the actuator in a third mode.
{Fig. 6B} Fig. 6B is a diagram showing voltage signals applied to the drive portion in the case in which the control portion of the scanning endoscope apparatus in Fig. 1 controls the actuator in the third mode.
{Fig. 7A} Fig. 7A is a diagram showing scanning tracks of the illumination light in the case in which the control portion of the scanning endoscope apparatus in Fig. 1 controls the actuator in a fourth mode.
{Fig. 7B} Fig. 7B is a diagram showing voltage signals applied to the drive portion in the case in which the control portion of the scanning endoscope apparatus in Fig. 1 controls the actuator in the fourth mode.
{Fig. 8} Fig. 8 is a schematic diagram of a scanning endoscope apparatus according to a second embodiment of the present invention.
{Fig. 9} Fig. 9 is a flowchart showing the pulling-back operation of the scanning endoscope apparatus in Fig. 8.
{Fig. 10} Fig. 10 is a flowchart showing a modification of the pulling-back operation of the scanning endoscope apparatus in Fig. 8.

### {Description of Embodiments}

A scanning endoscope apparatus according to a first embodiment of the present invention will be described below with reference to the drawings.

As shown in Figs. 1 and 2, a scanning endoscope apparatus 1 according to this embodiment is provided with a light source 2 that emits illumination light, a scanner 3 that two-dimensionally scans the illumination light emitted from the light source 2, a cylindrical apparatus main unit 4 that accommodates the scanner 3 in the interior thereof, a focusing lens 5 that is held at the distal-end side of the apparatus main unit 4 and that focuses the illumination light, a light detection portion 6 that receives return light (for example, reflected light or fluorescence) coming from an observation subject P and that detects the intensity of the return light, a speed detection portion 7 that detects the moving speed of the scanner 3, a direction detection portion 8 that detects the moving direction of the scanner 3, and a control portion 9 that controls the scanner 3.

As shown in Figs. 2 and 3, the scanner 3 is provided with an optical fiber 10 that guides the illumination light emitted from the light source 2 and that makes the illumination light exit from an exit end 11, a drive portion 12 that scans the illumination light exiting from the exit end 11 of the optical fiber 10, and a support portion 13 that supports the optical fiber 10.

As shown in Fig. 3, the drive portion 12 is provided with a rectangular-tube-shaped vibration transmitting member 15 that has a through-hole 14 in the axial direction through which the optical fiber 10 passes and four actuators 16 that are secured to four outer side surfaces 15a in the circumferential directions of the vibration transmitting member 15.

The actuators 16 are electromagnetic-type or piezo-type actuators, each of which has electrodes 17a and 17b provided on the two surfaces in the thickness direction thereof, as shown in Fig. 3, and, when driving voltages are applied to the electrodes 17a and 17b, vibrations are generated in directions and with amplitudes in accordance with the applied voltage signals. With the actuators 16, by securing the electrodes 17a to the external surfaces 15a of the vibration transmitting member 15 so that the directions of vibrations differ from each other by substantially 90°, the vibrations caused by the electrodes 17a and 17b are transmitted to the optical fiber 10 via the vibration transmitting member 15, and thus it is possible to make the exit end 11 of the optical fiber 10 vibrate in two axial directions, namely, X-axis and Y-axis directions, that intersect an optical axis S and that are substantially orthogonal thereto.

For example, by individually inputting to the actuators 16 voltage signals consisting of sine waves whose amplitudes linearly diminish and in which the phases thereof differ from each other by 90° about the optical axis, the exit end 11 of the optical fiber 10 is made to resonate, as shown in Fig. 4A, and thus, it is possible to radiate the illumination light in a spiraling manner onto the surface of the observation subject P.

As shown in Figs. 2 and 3, the support portion 13 is provided on the base-end side of the apparatus main unit 4 separated from the drive portion 12 in the longitudinal direction, and is a ring-like member that has a through-hole 18 in the thickness direction thereof along the longitudinal axis of the apparatus main unit 4. The optical fiber 10 is made to pass through the through-hole 18, thus being secured by the support portion 13, and an outer side surface 13a of the support portion 13 is in close contact with an inner side surface 4b of the apparatus main unit 4.

In addition, the support portion 13 secures the optical fiber 10 to the apparatus main unit 4 at a position closer to the base-end side thereof than the drive portion 12 is so as to suppress transmission of the vibrations transmitted to the optical fiber 10 from the drive portion 12 to the base-end side beyond the support portion 13.

As shown in Figs. 1 and 2, the light detection portion 6 is provided with a detection optical fiber 19 that receives return light coming from the observation subject P, generated by irradiation with the illumination light, and that guides the return light to the base-end side of the apparatus main unit 4, and a sensor 20 that detects the intensity of the return light guided thereto by the detection optical fiber 19.

As shown in Fig. 2, a plurality of the detection optical fibers 19 are arranged in the circumferential direction on an outer circumferential portion 4a of the apparatus main unit 4 so that entrance ends 21 thereof face forward.

The sensor 20 simultaneously receives the return light guided by the plurality of detection optical fibers 19 and detects the total intensity of the received return light. An image-processing portion (not shown) associates this return-light intensity with positions scanned by the light, thus generating an image.

As shown in Fig. 1, the speed detection portion 7 is provided in the outer circumferential portion 4a at an intermediate position of the apparatus main unit 4 in the longitudinal direction and is configured so as to detect the moving speed of the scanner 3 in the longitudinal direction, which moves together with the apparatus main unit 4.

The direction detection portion 8 is similarly provided in the outer circumferential portion 4a at the intermediate position of the apparatus main unit 4 in the longitudinal direction and is configured so as to detect the moving direction of the distal-end side or the base-end side along the longitudinal direction of the scanner 3 which moves together with the apparatus main unit 4.

The control portion 9 stores, as the voltage signals to be applied to the actuators 16, information about the voltage signals having waveforms shown in Figs. 4B to 6B. Thus, the control portion 9 is configured so as to apply the voltage signals to the actuators 16 by switching the voltage signals on the basis of the moving speed and the moving direction along the longitudinal direction of the scanner 3 that have been detected by the speed detection portion 7 and the direction detection portion 8.

Specifically, the control portion 9 stores information about voltage signals having a waveform that changes relatively slowly from a maximum amplitude to a minimum amplitude, as shown in Fig. 4B, voltage signals having a waveform that changes from the maximum amplitude to the minimum amplitude twice as fast as the case in Fig. 4B, as shown in Fig. 5B, and voltage signals having a waveform that changes from the maximum amplitude to a minimum amplitude greater than that in Fig. 4B at the same speed as the case in Fig. 4B, as shown in Fig. 6B.

Thus, in the case in which the moving direction along the longitudinal direction of the scanner 3 detected by the direction detection portion 8 is a forward direction with respect to the exiting direction of the illumination light and the speed along the longitudinal direction of the scanner 3 detected by the speed detection portion 7 is lower than a predetermined threshold, the voltage signals of Fig. 4B are output (first mode), and, in the case in which the detected speed is equal to or greater than the predetermined threshold, the voltage signals of Fig. 5B are output (second mode).

On the other hand, in the case in which the moving direction along the longitudinal direction of the scanner 3 detected by the direction detection portion 8 is a backward direction with respect to the exiting direction of the illumination light and the speed along the longitudinal direction of the scanner 3 detected by the speed detection portion 7 is lower than the predetermined threshold, the voltage signals of the first mode are output, and, in the case in which the detected speed is equal to or greater than the predetermined threshold, the voltage signals of Fig. 6B are output (third mode).

A control method of the thus-configured scanning endoscope apparatus 1 according to this embodiment will be described below.

In order to observe the observation subject P by using the scanning endoscope apparatus 1 according to this embodiment, in the state in which the exit end 11 of the optical fiber 10 is made to face the observation subject P, the control portion 9 supplies the voltage signals of Fig. 4B, which corresponds to the first mode, to the electrodes 17a and 17b of the actuators 16.

By doing so, the actuators 16 vibrate in the form corresponding to the supplied voltage signals, and the vibrations of the actuators 16 are transmitted to the optical fiber 10 via the vibration transmitting member 15. When the vibrations are transmitted to the optical fiber 10, the exit end 11 of the optical fiber 10 exhibits large vibrations in the two axial directions, namely, the X-axis and Y-axis directions, due to the resonation. When the illumination light emitted from the light source 2 is supplied to the optical fiber 10 in the state in which the exit end 11 is vibrating in the two axial directions, the illumination light is guided inside the optical fiber 10, exits from the exit end 11, and is two-dimensionally scanned over the observation subject P in accordance with the track shown in Fig. 4A.

Then, the return light that returns from the observation subject P due to the irradiation with the illumination light enters the entrance end 21 of the detection optical fiber 19 and is guided therethrough to be received by the sensor 20. By doing so, the image-processing portion associates the return-light intensities detected by the sensor 20 with the individual scanning positions of the optical fiber 10 when scanned by the scanner 3, and thus, generates an image of a return-light distribution.

As shown in Fig. 4B, because the scanning track of the illumination light of the first mode is generated by the voltage signals that change from the maximum amplitude to the minimum amplitude over a relatively long time, the scanning track takes a spiraling form having a relatively high density with relatively small spaces (pitches) B between adjacent scanning tracks, as shown in Fig. 4A. Thus, by acquiring the return light along the scanning track having the spiraling form, it is possible to acquire an image having a relatively high resolution.

Next, when a user moves the scanner 3 in the longitudinal direction of the scanner 3, the moving speed of the scanner 3 is detected by the speed detection portion 7 (speed detecting step), and the moving direction of the scanner 3 is detected by the direction detection portion 8. Then, in the case in which the moving direction detected by the direction detection portion 8 is a forward direction with respect to the exiting direction of the illumination light, and the moving speed detected by the speed detection portion 7 is equal to or greater than the threshold, the control portion 9 switches the voltage signals to the second mode shown in Fig. 5B (displacement adjusting step). By doing so, the voltage signals that change from the maximum amplitude to the minimum amplitude twice as fast as in the first mode are supplied to the actuators 16.

As shown in Fig. 5A, the scanning track of the illumination light of the second mode takes a spiraling form having a lower density than that of the first mode and having pitches C that are greater than the pitches B of the first mode. Accordingly, the scanning track of the second mode has a shorter length than that of the scanning track of the first mode.

Because the frequency for causing resonation in the optical fiber 10 does not change, in the second mode in which the scanning track is shorter, scanning is performed in less time from the maximum amplitude to the minimum amplitude, that is, from the outer circumference of the scanning track having the spiraling form to the center thereof. Thus, by acquiring the return light along the scanning track having the spiraling form, it is possible to acquire an image at a frame rate twice as great as that of the first mode.

In other words, in the second mode, it is possible to quickly acquire an image by suppressing the resolution so as to be lower than that of the first mode and by compensating therefor by increasing the frame rate. As a result, there is an advantage in that it is possible to decrease the image distortion even if the scanner 3 is moved at high speed.

On the other hand, in the case in which the moving direction detected by the direction detection portion 8 is a backward direction with respect to the exiting direction of the illumination light and the moving speed detected by the speed detection portion 7 is equal to or greater than the predetermined threshold, the control portion 9 switches the voltage signals to the third mode shown in Fig. 6B. By doing so, the voltage signals that change from the maximum amplitude to the minimum amplitude greater than that of the first mode at the same speed as in the first mode are supplied to the actuators 16.

As shown in Fig. 6A, the scanning track of the illumination light in the third mode takes a doughnut-type spiraling form in which the pitches B are the same as in the first mode and the area of a scanning region is decreased at the same density as in the first mode. By doing so, the length of the scanning track in the third mode is shorter than that of the first mode.

In the third mode in which the scanning track is shorter than that of the first mode, the scanning track having the doughnut-type spiraling form is scanned, in less time, from the outer circumference to the inner circumference, and the area near the center is not scanned. Thus, in the third mode also, by acquiring the return light along the scanning track, it is possible to acquire an image at a frame rate twice as great as in the first mode.

In other words, in the third mode, by limiting the scanning region to the vicinity of the periphery and by compensating therefor by increasing the frame rate, while maintaining the same resolution as in the first mode, it is possible to quickly acquire a high-quality image. As a result, there is an advantage in that it is possible to decrease the image distortion even if the scanner 3 is moved at high speed. In particular, in the case in which the observation subject P is an inner surface of the body cavity, and the viewing-field region of the scanner 3 is directed in the longitudinal direction of the body cavity or the like, because there is no return light coming from a center portion of the image, no information is lost even if the scanning region is limited to the peripheral portion, and thus, it is effective to enhance the frame rate.

Note that, in the scanning endoscope apparatus 1 according to this embodiment, although an example in which the control portion 9 controls the actuators 16 in the first mode when the moving speed of the scanner 3 in the longitudinal direction is lower than the threshold has been described, there is no limitation thereto. For example, as shown in Fig. 7B, the control portion 9 may supply the actuators 16 with voltage signals having a waveform that changes, at the same speed as in the first mode and at a greater density than that of the first mode, from the maximum amplitude to a minimum amplitude that is greater than that of the first mode (fourth mode).

As shown in Fig. 7A, the scanning track of the illumination light in the fourth mode takes a doughnut-type spiraling form in which pitches D are smaller than the pitches B of the first mode and the area of the scanning region is decreased at a greater density than that of the first mode. By doing so, the scanning track in the fourth mode has the same length as the scanning track of the first mode. As a result, without changing the scanning speed of the light, by compensating for a decrease in the area of the scanning region in the vicinity of the center of the scanning track by increasing the density of the scanning track at the outer circumference, it is possible to increase the resolution instead of decreasing the area of the viewing-field region.

In addition, in this embodiment, although the vibration transmitting member 15 is provided as the drive portion 12, alternatively, the actuators 16 may be secured to an external surface 10a of the optical fiber 10.

In addition, as the drive portion 12 in this embodiment, although the four actuators 16 are provided on the outer side surfaces 15a of the vibration transmitting member 15 to periodically vibrate the exit end 11 of the optical fiber 10 so as to cause displacement thereof in the two axial directions, there is no limitation thereto. For example, it suffices that two or more actuators 16 are provided.

In addition, in this embodiment, although an example in which the control portion 9 switches the lengths of the scanning track in accordance with the moving speed of the scanner 3 by using a single threshold has been described, the length of the scanning track may be continuously adjusted on the basis of the moving speed of the scanner 3 in the longitudinal direction. In addition, a plurality of thresholds may be set.

Next, a scanning endoscope apparatus 22 according to a second embodiment of the present invention will be described below with reference to the drawings.

In describing this embodiment, portions having the same configurations as those of the above-described scanning endoscope apparatus 1 according to the first embodiment will be given the same reference signs, and descriptions thereof will be omitted.

As shown in Fig. 8, the scanning endoscope apparatus 22 according to this embodiment differs from the scanning endoscope apparatus 1 according to the first embodiment in that a pulling-back means 23 (for example, a linear actuator) for pulling back the scanner 3 together with the apparatus main unit 4, a flushing portion 24 that discharges a flushing liquid in a forward direction with respect to the light-exiting direction when the apparatus main unit 4 is pulled back by the pulling-back means 23, and an image-recording portion (not shown) that records an acquired image are provided.

As shown in Fig. 8, the flushing portion 24 is provided at an intermediate position of the apparatus main unit 4 in the longitudinal direction in the form of a cylinder that is disposed so as to be separated radially outward from the apparatus main unit 4, and is provided with an injection port 25 that injects the flushing liquid that is a transparent liquid having biocompatibility (for example, physiological saline, low-molecular-weight dextran, or contrast medium) and a discharge portion 26 (for example, catheter) that discharges (flushes) the flushing liquid injected from the injection port 25 to the exterior of the flushing portion 24. The cylindrical gap between the flushing portion 24 and the apparatus main unit 4 is closed on the base-end side.

The injection port 25 is provided on the outside the intermediate position of the flushing portion 24 in the longitudinal direction so that the flushing liquid can be injected from the exterior into the gap between the flushing portion 24 and the apparatus main unit 4.

The discharge portion 26 is disposed on the distal-end side of the flushing portion 24, and is provided so as to discharge the flushing liquid injected into the gap between the flushing portion 24 and the apparatus main unit 4 in the forward direction.

As shown in Fig. 8, the pulling-back means 23 is provided in an outer circumferential portion 4a of the apparatus main unit 4 closer to the base-end side of the scanner 3 in the longitudinal direction than the flushing portion 24 is, and, when the user inputs a permission to pull back, the control portion 9 supplies the drive signals so that the scanner 3 is moved in a backward direction with respect to the light-exiting direction together with the apparatus main unit 4 (pulling-back operation).

The image-processing portion processes the acquired image, and detects the discharge of the flushing liquid on the basis of the changes in that image. The discharge of the flushing liquid can easily be detected on the basis of changes in the contrast of the image or the like.

In the case in which the discharge of the flushing liquid is detected by the image-processing portion in the state in which the permission to pull back has been input by the user, the control portion 9 switches the control so that the actuators 16 are controlled in the third mode, and also supplies signals to the image-recording portion to start recording images. Thus, the control portion 9 starts the pulling-back operation in the recording state, stops the recording by the image-recording portion when the pulling-back operation is completed, and switches the control so that the actuators 16 are controlled in the first mode.

The operation of the thus-configured scanning endoscope apparatus 22 according this embodiment will be described below.

Fig. 9 shows a flowchart for the scanning endoscope apparatus 22 according to this embodiment.

In a lumen (for example, blood vessel or the like), which is the observation subject P, filled with an opaque liquid, the control portion 9 controls the actuators 16 in the first mode to observe the observation subject P, as shown in Fig. 8. The user connects the pulling-back means 23 with the apparatus main unit 4 in the state in which the apparatus main unit 4 has reached a predetermined position. Then, the user permits pulling back by the pulling-back means 23 when a state in which pulling back can be performed has been achieved. When the user injects the flushing liquid from the injection port 25 in the state in which pulling back is permitted, the injected flushing liquid is discharged into the lumen from the discharge portion 26. Thus, when the discharge of the flushing liquid into the lumen is detected by means of the image processing by the image-processing portion, the control portion 9 switches the control of the actuators 16 to the third mode.

Then, once the control of the actuators 16 is switched to the third mode, the control portion 9 causes the image-recording portion to start recording images of the observation subject P and supplies the drive signals to the pulling-back means 23, thus starting the pulling-back operation of the scanner 3 and the apparatus main unit 4. At this time, because the control portion 9 controls the actuators 16 in the third mode, images without distortion are acquired, even if the apparatus main unit 4 is moved at high speed by the pulling-back means 23. Once the apparatus main unit 4 is pulled back by a predetermined distance, the control portion 9 stops the pulling-back operation, stops recording by the image-recording portion, and switches the control of the actuators 16 to the first mode until flushing is detected again.

By doing so, in the lumen, by discharging the flushing liquid from the discharge portion 26 while pulling back the apparatus main unit 4, because it is possible to make the exit end 11 of the optical fiber 10 pass through a region in which the discharged flushing liquid is temporarily retained, it is possible to acquire a clear image of the observation subject P by temporarily ensuring a sufficient viewing field.

In addition, because discharge of the flushing liquid is detected by using an image of a large viewing-field region acquired in the first mode, it is possible to accurately detect a state in which the interior of the lumen is uniformly flushed.

In addition, with the scanning endoscope apparatus 22 according to the embodiment described above, although an example controlled in accordance with the flowchart shown in Fig. 9 has been described, alternatively, the scanning endoscope apparatus 22 may be controlled in accordance with the flowchart shown in Fig. 10.

In this case, the control portion 9 controls the actuators 16 in the first mode. Once the user permits pulling back by the pulling-back means 23, the control portion 9 switches the control of the actuators 16 to the third mode.

Then, when the user injects the flushing liquid from the injection port 25 to discharge the flushing liquid into the lumen, the discharge of the flushing liquid in the lumen is detected by means of the image processing, and the control portion 9 causes the image-recording portion to start recording images and supplies the drive signals to the pulling-back means 23, thus starting the pulling-back operation of the scanner 3 and the apparatus main unit 4. The control portion 9 stops the pulling-back operation when the apparatus main unit 4 is pulled back by the predetermined distance, stops the recording by the image-recording portion, and maintains the actuators 16 in the third mode until flushing is detected again.

In addition, in this embodiment, when the scanning endoscope apparatus 1 or 22 performs the image processing by means of the image-processing portion, the regions that are not scanned in the image may be colored in colors other than black, or edges of the image may be colored.

By doing so, the user can easily distinguish the scanned regions and the non-scanned regions during observation.

In addition, in the individual embodiments described above, although examples in which the scanning tracks have a spiraling form have been described, alternatively, scanning tracks having a zigzag form based on the raster scanning method may be employed. By doing so also, it is possible to acquire a low-distortion image even during high-speed movement by increasing the frame rate instead of decreasing the resolution by increasing the pitches between the tracks by decreasing the length of a scanning track for manipulating one picture.

### {Reference Signs List}

- 1, 22: scanning endoscope apparatus
- 2: light source
- 3: scanner
- 6: light detection portion
- 7: speed detection portion
- 8: direction detection portion
- 9: control portion
- 10: optical fiber
- 11: optical-fiber exit end
- 12: drive portion
- 24: flushing portion

## Claims

1. A scanning endoscope apparatus (1; 22) comprising:
a scanner (3) that has an optical fiber (10), which is configured to guide light coming from a light source (2) and to make the light exit from an exit end (11) thereof, and a drive portion (12), which is configured to displace the exit end (11) of the optical fiber (12) in a direction that intersects an optical axis, and that is configured to scan, in a spiraling manner, the light that is made to exit from the exit end (11) over an observation subject (P);
a light detection portion (6) that is configured to receive return light coming from the observation subject (P), generated by the scanning of the light by the scanner (3);
a speed detection portion (7) that is configured to detect a moving speed of the scanner (3) relative to the observation subject; and
a control portion (9) that is configured to control the drive portion (12) to decrease, with an increase in the moving speed detected by the speed detection portion (7), a length of a scanning track of the light on the observation subject (P), **characterised in that**
the scanning track takes a doughnut-type spiraling form in which the pitches are maintained, so that the scanning region is limited to the peripheral portion and the area near the center is not scanned, whereby an area of the scanning region is decreased.

2. A scanning endoscope apparatus (1; 22) according to Claim 1, wherein the control portion (9) is configured to switch to control the drive portion to decrease the length of the scanning track when the moving speed detected by the speed detection portion (7) becomes equal to or greater than a predetermined threshold.

3. A scanning endoscope apparatus (1; 22) according to Claim 1 or 2,
wherein the drive portion (12) is configured to cause the exit end (11) to periodically vibrate, and
the control portion controls (9) the drive portion (12) so as not to change a pitch of the scanning track when changing the length of the scanning track.

4. A scanning endoscope apparatus (1; 22) according to Claim 1 or 2, further comprising:
a direction detection portion (8) that is configured to detect moving direction of the scanner (3),
wherein the control portion (9) is configured to perform the control of the drive portion (12) to change the area of the scanning region only when the moving direction detected by the direction detection portion (8) is a backward direction with respect to the exiting direction in which the light exits from the exit end (11).

5. A scanning endoscope apparatus (1; 22) according to Claim 4,
wherein the control portion (9) is configured to control the drive portion (12) to change a density of the scanning track in a predetermined scanning region when the moving direction detected by the direction detection portion (8) is a forward direction with respect to the exiting direction in which the light exits from the exit end (11).

6. A scanning endoscope apparatus (1; 22) according to Claim 4 or 5, further comprising:
a flushing portion (24) that is provided in an outer circumferential portion of the scanner (3) at an intermediate position thereof in a longitudinal direction and that is configured to discharge a transparent liquid in a forward direction with respect to the light-exiting direction when the scanner (3) is moved in a backward direction with respect to the light-exiting direction.

## Patentansprüche

1. Endoskopische Abtastvorrichtung (1; 22), umfassend:
einen Abtaster (3), der eine optische Faser (10) aufweist, die dazu ausgelegt ist, von einer Lichtquelle (2) kommendes Licht zu leiten und dafür zu sorgen, dass das Licht aus einem Austrittsende (11) davon austritt, und einen Antriebsabschnitt (12), der dazu ausgelegt ist, das Austrittsende (11) der optischen Faser (12) in einer Richtung zu verlagern, die ein optische Achse schneidet, und der dazu ausgelegt ist, das Licht, das dazu gebracht wird, am Austrittsende (11) auszutreten, über einem Beobachtungssubjekt (P) spiralförmig abzutasten;
einen Lichterfassungsabschnitt (6), der dazu ausgelegt ist, von dem Beobachtungssubjekt (P) kommendes Rückkehrlicht zu empfangen, das durch das Abtasten des Lichts durch den Abtaster (3) erzeugt wurde;
einen Geschwindigkeitserfassungsabschnitt (7), der dazu ausgelegt ist, eine Bewegungsgeschwindigkeit des Abtasters (3) bezogen auf das Beobachtungssubjekt zu erfassen; und
einen Steuerungsabschnitt (9), der dazu ausgelegt ist, den Antriebsabschnitt (12) zu steuern, um bei einem Anstieg in der von dem Geschwindigkeitserfassungsabschnitt (7) erfassten Bewegungsgeschwindigkeit eine Länge einer Abtastspur des Lichts an dem Beobachtungssubjekt (P) zu verringern
**dadurch gekennzeichnet, dass**
die Abtastspur eine Donut-artige Spiralform annimmt, bei der die Abstände beibehalten werden, sodass die Abtastregion auf den peripheren Abschnitt begrenzt ist und die Fläche nahe der Mitte nicht abgetastet wird, wodurch eine Fläche der Abtastregion verringert wird.

2. Endoskopische Abtastvorrichtung (1; 22) nach Anspruch 1, wobei der Steuerungsabschnitt (9) dazu ausgelegt ist, zu schalten, um den Antriebsabschnitt zu steuern, um die Länge der Abtastspur zu verringern, wenn die von dem Geschwindigkeitserfassungsabschnitt (7) erfasste Bewegungsgeschwindigkeit gleich oder größer wird als ein vorbestimmter Schwellenwert.

3. Endoskopische Abtastvorrichtung (1; 22) nach Anspruch 1 oder 2,
wobei der Antriebsabschnitt (12) dazu ausgelegt ist, zu bewirken, dass das Austrittsende (11) periodisch vibriert, und
der Steuerungsabschnitt (9) den Antriebsabschnitt (12) derart steuert, dass kein Abstand der Abtastspur geändert wird, wenn die Länge der Abtastspur geändert wird.

4. Endoskopische Abtastvorrichtung (1; 22) nach Anspruch 1 oder 2, ferner umfassend:
einen Richtungserfassungsabschnitt (8), der dazu ausgelegt ist, eine Bewegungsgeschwindigkeit des Abtasters (3) zu erfassen,
wobei der Steuerungsabschnitt (9) dazu ausgelegt ist, die Steuerung der Steuerung des Antriebsabschnitts (12) durchzuführen, um die Fläche der Abtastregion nur zu verändern, wenn die von dem Richtungserfassungsabschnitt (8) erfasste Bewegungsrichtung eine Rückwärtsrichtung mit Bezug auf die Austrittsrichtung ist, in der das Licht aus dem Austrittsende (11) austritt.

5. Endoskopische Abtastvorrichtung (1; 22) nach Anspruch 4,
wobei der Steuerungsabschnitt (9) dazu ausgelegt ist, den Antriebsabschnitt (12) zu steuern, um eine Dichte der Abtastspur in einer vorbestimmten Abtastregion zu ändern, wenn die von dem Richtungserfassungsabschnitt (8) erfasste Bewegungsrichtung eine Vorwärtsrichtung mit Bezug auf die Austrittsrichtung ist, in der das Licht aus dem Austrittsende (11) austritt.

6. Endoskopische Abtastvorrichtung (1; 22) nach Anspruch 4 oder 5, ferner umfassend:
einen Spülabschnitt (24), der in einem Außenumfangsabschnitt des Abtasters (3) an einer Zwischenposition davon in einer Längsrichtung vorgesehen und dazu ausgelegt ist, eine transparente Flüssigkeit in einer Vorwärtsrichtung mit Bezug auf die Lichtaustrittsrichtung abzuführen, wenn der Abtaster (3) in eine Rückwärtsrichtung mit Bezug auf die Lichtaustrittsrichtung bewegt wird.

## Revendications

1. Appareil endoscopique à balayage (1 ; 22) comprenant :
un dispositif de balayage (3) qui comprend une fibre optique (10), qui est configurée pour guider de la lumière provenant d'une source de lumière (2) et pour amener la lumière à sortir par une extrémité de sortie (11) de celle-ci, et une partie d'entraînement (12), qui est configurée pour déplacer l'extrémité de sortie (11) de la fibre optique (12) dans une direction qui coupe un axe optique, et qui est configuré pour balayer, en spirale, la lumière qui est amenée à sortir par l'extrémité de sortie (11) au-dessus d'un sujet d'observation (P) ;
une partie de détection de lumière (6) qui est configurée pour recevoir une lumière de retour provenant du sujet d'observation (P), générée par le balayage de la lumière par le dispositif de balayage (3) ;
une partie de détection de vitesse (7) qui est configurée pour détecter une vitesse de déplacement du dispositif de balayage (3) par rapport au sujet d'observation ; et
une partie de commande (9) qui est configurée pour commander la partie d'entraînement (12) afin de diminuer, pour une augmentation de la vitesse de déplacement détectée par la partie de détection de vitesse (7), une longueur d'un trajet de balayage de la lumière sur le sujet d'observation (P),
**caractérisé par le fait que** le trajet de balayage prend une forme de spirale de type anneau dans laquelle les pas sont maintenus, de telle sorte que la région de balayage est limitée à la partie périphérique et la surface zone à proximité du centre n'est pas balayée, ce par quoi une surface de la région de balayage étant diminuée.

2. Appareil endoscopique à balayage (1 ; 22) selon la revendication 1, dans lequel la partie de commande (9) est configurée pour commuter afin de commander la partie d'entraînement de façon à diminuer la longueur du trajet de balayage lorsque la vitesse de déplacement détectée par la partie de détection de vitesse (7) devient égale ou supérieure à un seuil prédéterminé.

3. Appareil endoscopique à balayage (1 ; 22) selon la revendication 1 ou 2,
dans lequel la partie d'entraînement (12) est configurée pour amener l'extrémité de sortie (11) à vibrer de manière périodique, et
la partie de commande (9) commande la partie d'entraînement (12) de façon à ne pas changer un pas du trajet de balayage lors d'un changement de la longueur du trajet de balayage.

4. Appareil endoscopique à balayage (1 ; 22) selon la revendication 1 ou 2, comprenant en outre :
une partie de détection de direction (8) qui est configurée pour détecter une direction de déplacement du dispositif de balayage (3),
la partie de commande (9) étant configurée pour réaliser la commande de la partie d'entraînement (12) de façon à changer la surface de la région de balayage seulement lorsque la direction de déplacement détectée par la partie de détection de direction (8) est une direction vers l'arrière par rapport à la direction de sortie dans laquelle la lumière sort de l'extrémité de sortie (11).

5. Appareil endoscopique à balayage (1 ; 22) selon la revendication 4,
dans lequel la partie de commande (9) est configurée pour commander la partie d'entraînement (12) de façon à changer une densité du trajet de balayage dans une région de balayage prédéterminée lorsque la direction de déplacement détectée par la partie de détection de direction (8) est une direction vers l'avant par rapport à la direction de sortie dans laquelle la lumière sort de l'extrémité de sortie (11).

6. Appareil endoscopique à balayage (1 ; 22) selon la revendication 4 ou 5, comprenant en outre :
une partie de rinçage (24) qui est prévue dans une partie circonférentielle externe du dispositif de balayage (3) à une position intermédiaire de celui-ci dans une direction longitudinale et qui est configurée pour déverser un liquide transparent dans une direction vers l'avant par rapport à la direction de sortie de lumière lorsque le dispositif de balayage (3) est déplacé dans une direction vers l'arrière par rapport à la direction de sortie de lumière.
